# EUROPEAN PATENT APPLICATION

(11) **EP 4 427 730 A1**
(43) Date of publication of application: **11.09.2024**
(21) Application number: 24159860.6
(22) Date of filing: 27.02.2024
(51) Int. Cl.: A61K 8/58, A61K 8/891, A61K 8/895, A61Q 1/00, A61Q 1/02, A61Q 1/06, A61Q 1/12, A61Q 17/00

(54) **COSMETIC COMPRISING AN ORGANOSILOXANE**

(30) Priority: 10.03.2023 JP 2023037615
(71) Applicant: SHIN-ETSU CHEMICAL CO., LTD., Tokyo 1000005 (JP)
(72) Inventor: Mizuno, Akiko, Tokyo (JP)
(74) Representative: Sonnenhauser, Thomas Martin

(57) **Abstract**

The inventive cosmetic includes; (A) organosiloxane represented by the following formula (1), and having a boiling point in a range of 205 to 255°C and a kinematic viscosity at 25°C of less than 5 mm²/s; and (B) volatile oil having a kinematic viscosity at 25°C of 2 mm²/s or less, excluding the component (A). 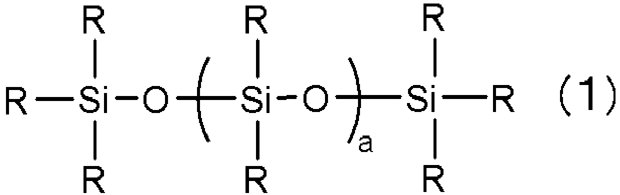 wherein, R each independently may be identical to or different from one another and represents a group selected from the group consisting of a hydrogen group, a hydroxy group, and a monovalent hydrocarbon group having 1 to 3 carbon atoms. "a" is an integer from 1 to 5. It is provided that at least one of R is a monovalent hydrocarbon group having 2 or 3 carbon atoms. This cosmetic can provide a light feeling, good spreadability and a uniform cosmetic film, and exhibit excellent stability over time and excellent cosmetic durability.

## Description

### TECHNICAL FIELD

The present invention relates to a cosmetic containing organosiloxane.

### BACKGROUND ART

Silicones typified by dimethylpolysiloxane have features such as a light touch, good spreadability, excellent water-repellency, and high safety, and therefore in recent years, silicones have been widely used as an oil agent in cosmetics.

For example, Patent Document 1 discloses that a cosmetic containing a volatile cyclic siloxane such as octamethylcyclotetrasiloxane (D4), decamethylcyclopentasiloxane (D5), dodecamethylcyclohexasiloxane (D6), etc. exhibits a light feeling when applied to the skin, good spreadability, and excellent water-repellency.

In recent years, in certain cases, linear dimethyl silicone oil having a kinematic viscosity of 2 mm²/s or less (at 25°C) has been studied as an alternative to cyclic silicone. However, the usability and stability of the cosmetic deteriorate in some cases, because there are cases where the dimethyl silicone oil has poor compatibility with a polar oil agent and thus cannot be mixed therewith in a clear state.

On the other hand, it is also known that a phenyl-modified silicone, long-chain alkyl-modified silicone and the like are used to enhance the compatibility with a polar oil agent. However, such silicones are less volatile than cyclic siloxanes, and therefore, they lose their light feeling when applied to the skin, and in makeup cosmetics, they are slow to complete forming of coating-film and are prone to cause color migration in some cases.

Meanwhile, for the purpose of providing an alternative to formulate a composition containing volatile oils, Patent Document 2 proposes a composition which contains at least one non-cyclic volatile silicone oil and has a specific evaporation profile. However, it is impossible to find out a description on formulation embodiments other than decamethyltetrasiloxane and dodecamethylpentasiloxane in the document, and the document does not consider the usability nor the stability of cosmetics.

### CITATION LIST

### PATENT LITERATURE

Patent Document 1: WO 2001/015658 A1
Patent Document 2: WO 2004/087077 A1

### SUMMARY OF INVENTION

### TECHNICAL PROBLEM

The present invention has been made in view of the above circumstances. An object of the present invention is to provide a cosmetic which can provide a light feeling, good spreadability and a uniform cosmetic film, and exhibit excellent stability over time and excellent cosmetic durability.

### SOLUTION TO PROBLEM

To solve the above problems, the present invention provides a cosmetic comprising:
(A) organosiloxane represented by the following formula (1), and having a boiling point in a range of 205 to 255°C and a kinematic viscosity at 25°C of less than 5 mm²/s, 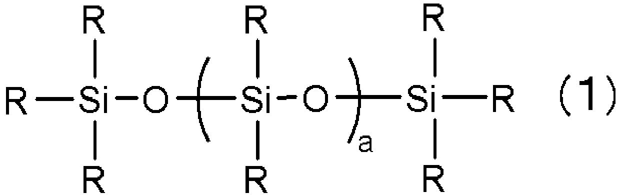 wherein R each independently may be identical to or different from one another and represents a group selected from the group consisting of a hydrogen group, a hydroxy group, and a monovalent hydrocarbon group having 1 to 3 carbon atoms, and "a" is an integer from 1 to 5, provided that at least one of R is a monovalent hydrocarbon group having 2 or 3 carbon atoms; and
(B) volatile oil having a kinematic viscosity at 25°C of 2 mm²/s or less, excluding the component (A).

The inventive cosmetic includes the organosiloxane(s) shown by the general formula (1) as the component (A) in a base composition, and thus, can provide a light feeling, good spreadability, excellent water repellency, and a uniform cosmetic film. In addition, it is possible to provide the cosmetic which is excellent in stability over time and cosmetic durability even in a mixture system with various oil agents such as silicone oil, hydrocarbon oil, ester oil, etc., an oil-based component being solid at 25°C, or an organic ultraviolet absorber. Further, by virtue of mixing with volatile oil, which is different from the component (A) and has a kinematic viscosity at 25°C of 2 mm²/s or less, as the component (B), the light feeling is not impaired even when containing the above oil-based component. Furthermore, the completion of coating film can be accelerated, and it is possible to obtain makeup cosmetics with the difficulty of color migration.

That is the inventive cosmetic can provide a light feeling, good spreadability, excellent water repellency, and a uniform cosmetic film, and further can achieve favorable usability without giving a strong oily feeling. Furthermore, even when containing a polar oil agent, the inventive cosmetic can exhibit excellent stability over time and excellent cosmetic durability.

In this event, in the formula (1) of the organosiloxane as the component (A), "a" is preferably 2.

The cosmetic containing such an organosiloxane can achieve more favorable usability without giving a strong oily feeling and can have excellent stability over time and excellent cosmetic durability even when containing a polar oil agent.

Further, it is more preferable that the organosiloxane as the component (A) is a compound selected from the group consisting of 3,5-diethyl-1,1,1,3,5,7,7,7-octamethyltetrasiloxane, 3,3-diethyl-1,1,1,5,5,7,7,7-octamethyltetrasiloxane, 1,3-diethyl-1,1,3,5,5,7,7,7-octamethyltetrasiloxane, 1,5-diethyl-1,1,3,3,5,7,7,7-octamethyltetrasiloxane, 1,7-diethyl-1,1,3,3,5,5,7,7-octamethyltetrasiloxane, 3,5-dipropyl-1,1,1,3,5,7,7,7-octamethyltetrasiloxane, 3,3-dipropyl-1,1,1,5,5,7,7,7-octamethyltetrasiloxane, 1,3-dipropyl-1,1,3,5,5,7,7,7-octamethyltetrasiloxane, 1,5-dipropyl-1,1,3,3,5,7,7,7-octamethyltetrasiloxane, 1,7-dipropyl-1,1,3,3,5,5,7,7-octamethyltetrasiloxane, and 3-ethyl-5-propyl-1,1,1,3,5,7,7,7-octamethyltetrasiloxane.

Such organosiloxane is excellent in compatibility with an organic ultraviolet absorber. Therefore, cosmetics containing such organosiloxane can more surely achieve favorable usability without giving strong oily feeling, and can exhibit excellent stability over time and excellent cosmetic durability even when containing a polar oil agent, especially with an organic ultraviolet absorber.

Further, in the inventive cosmetic, the volatile oil as the component (B) is preferably at least one compound selected from the group consisting of linear dimethyl silicone oils having a kinematic viscosity at 25°C of less than 2 mm²/s and methyl trimethicone.

The linear dimethyl silicone oils having a kinematic viscosity at 25°C of less than 2 mm²/s or the methyl trimethicone are excellent in a light feeling, good spreadability, and water repellency when applied to the skin, and thus the cosmetic can exhibit favorable usability, excellent stability over time and excellent cosmetic durability.

Additionally, it is preferable that the inventive cosmetic further contains one or more (C) silicone surfactants having a polyether group or a polyglycerin group.

Such cosmetic can exhibit more favorable usability, more excellent stability over time, and more excellent cosmetic durability.

### ADVANTAGEOUS EFFECTS OF INVENTION

As described above, the inventive cosmetic can provide a light feeling, good spreadability, and a uniform cosmetic film, and exhibit excellent stability over time and excellent cosmetic durability.

### DESCRIPTION OF EMBODIMENTS

As noted above, it has been desired to develop cosmetics which can provide a light feeling, good spreadability, uniform cosmetic film, and exhibit excellent stability over time and excellent cosmetic durability.

As a result of their diligent study of the above problems, the inventor found that a cosmetic containing organosiloxane having specific structure as a component (A) and volatile oil which is different from the component (A) and has a kinematic viscosity at 25°C of 2 mm²/s or less as a component (B) can provide a light feeling, good spreadability, excellent water repellency and a uniform cosmetic film, and exhibit excellent stability over time and excellent cosmetic durability, and then have completed the present invention.

That is, the present invention relates to cosmetic comprising (A) organosiloxane represented by the following formula (1), and having a boiling point in a range of 205 to 255°C and a kinematic viscosity at 25°C of less than 5 mm²/s, 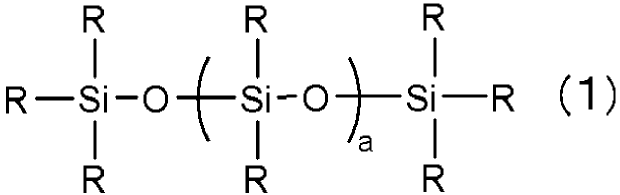 wherein R each independently may be identical to or different from one another and represents a group selected from the group consisting of a hydrogen group, a hydroxy group, and a monovalent hydrocarbon group having 1 to 3 carbon atoms, and "a" is an integer from 1 to 5, provided that at least one of R is a monovalent hydrocarbon group having 2 or 3 carbon atoms; and
(B) volatile oil having a kinematic viscosity at 25°C of 2 mm²/s or less, excluding the component (A).

Hereinafter, the present invention is described in detail, however, the present invention is not limited thereto.

Herein, component names may be written according to Japanese Labeling Name of Cosmetic Ingredient (hereinafter, Labeling Name) or International Nomenclature of Cosmetic Ingredients (INCI). Regarding component having Japanese Labeling Name corresponding to that of INCI, the English description of the Labeling Name may be omitted.

The inventive cosmetic comprises:
(A) organosiloxane represented by the above formula (1), and having a boiling point in a range of 205 to 255°C and a kinematic viscosity at 25°C of less than 5 mm²/s; and
(B) volatile oil having a kinematic viscosity at 25°C of 2 mm²/s or less, excluding the component (A).

By virtue of including organosiloxane having the above specific structure as the component (A) and volatile oil different from the component (A) and having a kinematic viscosity at 25°C of 2 mm²/s or less as the component (B), the inventive cosmetic can provide have a light feeling, good spreadability, excellent water repellency, and a uniform cosmetic film, and can exhibit excellent stability over time and cosmetic durability without impairing a light feeling. Furthermore, the completion of coating film can be accelerated, and it is possible to obtain makeup cosmetics with the difficulty of color migration.

The inventive cosmetic can further contain optional components other than the components (A) and (B).

Essential components and optional components of the inventive cosmetic are described below. It should be noted that examples of optional components are listed in the latter section, but examples that meet the definition of the component (A) or (B), which are essential components, shall be included in the component (A) or (B) the definition of which the examples meet.

### [Component (A)]

The component (A) which is an essential component for the inventive cosmetic is organosiloxane which is represented by the following formula (1) and has a boiling point in a range of 205 to 255°C and a kinematic viscosity at 25°C of less than 5 mm²/s, 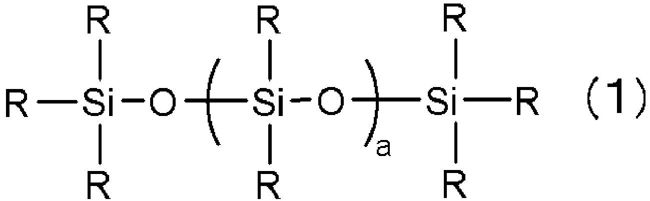 wherein, R each independently may be identical to or different from one another and represents a group selected from the group consisting of a hydrogen group, a hydroxy group, and a monovalent hydrocarbon group having 1 to 3 carbon atoms, and "a" is an integer from 1 to 5, provided that at least one of R is a monovalent hydrocarbon group having 2 or 3 carbon atoms.

In the above formula (1), each R each independently may be identical to or different from one another, and represents a group selected from the group consisting of a hydrogen group, a hydroxy group, and a monovalent hydrocarbon group having 1 to 3 carbon atoms. Examples of the monovalent hydrocarbon group include a methyl group, an ethyl group, and a propyl group. Provided that at least one of R is a monovalent hydrocarbon group having 2 or 3 carbon atoms, specific examples thereof include an ethyl group and a propyl group.

In the above formula (1), "a" is preferably 2. Cosmetic including such organosiloxane can achieve a more favorable usability without giving a strong oily feeling and exhibit excellent stability over time and excellent cosmetic durability even when blended with a polar oil agent.

Because the (A) organosiloxane in the present invention has a boiling point in a range of 205 to 255°C and is volatile, the inventive cosmetic does not give a strong oily feeling after applying it to the skin. When the boiling point is lower than 205°C, it becomes difficult to have good spreadability. When the boiling point is higher than 255°C, the cosmetic gives a strong oily feeling. In the present invention, the boiling point refers to the boiling point under an atmospheric pressure of 1013 hPa.

Furthermore, because the (A) organosiloxane in the present invention has a kinematic viscosity at 25°C of less than 5 mm²/s, the inventive cosmetic can have good spread feeling similarly as a case using decamethylcyclopentasiloxane having a kinematic viscosity of 4 mm²/s (at 25°C) or the like, and can give a favorable usability without giving a significant sticky feeling. When the kinematic viscosity at 25°C is 5 mm²/s or higher, the cosmetic is poor in usability due to poor spreadability and a sticky feeling. It should be noted that in the present invention, the kinematic viscosity is a value measured at 25°C by using a Cannon-Fenske viscometer described in JIS Z 8803:2011.

The organosiloxane as the component (A) of the present invention is preferably a compound selected from the group consisting of 3,5-diethyl-1,1,1,3,5,7,7,7-octamethyltetrasiloxane, 3,3-diethyl-1,1,1,5,5,7,7,7-octamethyltetrasiloxane, 1,3-diethyl-1,1,3,5,5,7,7,7-octamethyltetrasiloxane, 1,5-diethyl-1,1,3,3,5,7,7,7-octamethyltetrasiloxane, 1,7-diethyl-1,1,3,3,5,5,7,7-octamethyltetrasiloxane, 3,5-dipropyl-1,1,1,3,5,7,7,7-octamethyltetrasiloxane, 3,3-dipropyl-1,1,1,5,5,7,7,7-octamethyltetrasiloxane, 1,3-dipropyl-1,1,3,5,5,7,7,7-octamethyltetrasiloxane, 1,5-dipropyl-1,1,3,3,5,7,7,7-octamethyltetrasiloxane, 1,7-dipropyl-1,1,3,3,5,5,7,7-octamethyltetrasiloxane, and 3-ethyl-5-propyl-1,1,1,3,5,7,7,7-octamethyltetrasiloxane. Such organosiloxane is excellent in compatibility with an organic ultraviolet absorber. Therefore, cosmetic including such organosiloxane can more surely achieve a more favorable usability without giving a strong oily feeling, and exhibit excellent stability over time and excellent cosmetic durability even when containing a polar oil agent, especially an organic ultraviolet absorber.

As the (A) organosiloxane of the present invention, a commercially available product may be used. Specific examples include KF-4422 manufactured by Shin-Etsu Chemical Co., Ltd.

The (A) organosiloxane of the present invention can be used in combination with any component by virtue of being excellent in compatibility with general purpose ingredients which can be contained in a variety of cosmetics. Especially, the (A) organosiloxane is excellent in compatibility with an organic ultraviolet absorber such as ethylhexyl methoxycinnamate. Further, in case of containing silicone to have a light usability, the (A) organosiloxane can produce cosmetics excellent in stability over time and excellent cosmetic durability.

Furthermore, when used in combination with an oil-based component being solid at 25°C, stick-like formulation can be easily prepared without interfering with the solidification of the oil-based component. Examples of oily components being solid at 25°C include polyethylene, ceresin, ozokerite, beeswax, microcrystalline wax, stearyl alcohol, behenyl alcohol, cetanol, etc.

As described above, by virtue of using the (A) organosiloxane of the present invention, the cosmetic can provide a light feeling, good spreadability, excellent water repellency, and a uniform cosmetic film, and further can achieve favorable usability without giving a strong oily feeling. Furthermore, even in a mixture system with various oil agents such as an organic ultraviolet absorber, silicone, hydrocarbon oil, ester oil, or with an oil-based component being solid at 25°C, the cosmetics can be excellent in stability over time and cosmetic durability. Additionally, stick-like formulation can also be easily prepared.

For the (A) organosiloxane of the present invention, usability and volatilization rate can also be adjusted by combination of two or more kinds which are different in kinematic viscosity. For the purpose of achieving moderate volatilization rate, strict adjustment is not required, and one kind can be also used solely for the cosmetic.

The content of the component (A) is not specifically limited, but is preferably 1 to 70 mass% relative to the mass of the entire cosmetic.

### [Component (B)]

The component (B) of the present invention is volatile oil having a kinematic viscosity at 25°C of 2 mm²/s or less, provided that the component (B) excludes organosiloxane as the component (A). In the present invention, volatile oil refers to oil having a boiling point of 250°C or less under an atmospheric pressure of 1013 hPa.

A cosmetic which contains the component (A) but does not contain volatile oil having a kinematic viscosity at 25°C of 2 mm²/s or less cannot exhibit both properties of a light feeling and excellent cosmetic durability.

The component (B) is not limited as long as it is volatile oil having a kinematic viscosity at 25°C of 2 mm²/s or less, and one kind of oil can be used solely or the mixture of two kinds or more can be appropriately used. The specific examples thereof include oil-based volatile component such as dimethylpolysiloxane (KF-96A-1cs, KF-96L-1.5cs, KF-96L-2cs, and the like; manufactured by Shin-Etsu Chemical Co., Ltd.) having a boiling point of 250°C or less, tris(trimethylsiloxy)methylsilane (TMF-1.5; manufactured by Shin-Etsu Chemical Co., Ltd.), light isoparaffin, undecane, isododecane. Among these, linear dimethylsilicone oil having a kinematic viscosity of less than 2 mm²/s is most preferable in terms of versatility and usability. The linear dimethylsilicone oil having a kinematic viscosity of less than 2 mm²/s is more volatile than the component (A), and thus the cosmetic having adjusted volatilization rate and difficulty of color migration can be obtained. The other preferable examples of the component (B) can include methyl trimethicone.

The inventive cosmetic may contain, as the component (B), one kind of volatile oil or two or more kinds of volatile oil having different kinematic viscosity.

The content of the component (B) is preferably not less than 1 mass% and not more than 70 mass%, more preferably not less than 1 mass% and not more than 50 mass%, further preferably not more than 30 mass%, relative to the mass of the entire cosmetic.

Further, in the present cosmetic, the mass of the volatile oil as the component (B) (when containing two or more kinds, the total sum thereof) is preferably not less than 0.1% and not more than 6%, more preferably not less than 0.2% and not more than 4%, relative to mass of organopolysiloxane as the component (A). When the mass of volatile oil as the component (B) relative to the mass of organosiloxane as the component (A) is within the preferable range, the cosmetic durability is especially excellent.

### [Component (C)]

The inventive cosmetic preferably contain one or more silicone surfactants having a polyether group or a polyglycerin group as a component (C) as well as the components (A) and (B). The silicone surfactants are, for example, one or more compounds selected from the group consisting of crosslinked silicone surfactants and noncrosslinked silicone surfactants. The component (C) can exhibit good compatibility with components (A) and (B), and can make a cosmetic stable even when an organic ultraviolet absorber is contained. Especially when a noncrosslinked silicone surfactant or a crosslinked silicone surfactant each having an alkyl branch on a silicone main chain is used, the cosmetic is more stable.

The content of the component (C) is preferably in the range of 0.1 to 20 mass% relative to the mass of the entire cosmetic. When contained in 0.1 mass% or more, the silicone surfactant can perform functions of dispersion or emulsification sufficiently. When contained in 20 mass% or less, the cosmetic cannot exhibit a sticky touch on use, so it is preferable. The HLB value of the silicone surfactant is not limited, but it is preferable in the range of 2 to 14.5 to maintain water resistance of the cosmetic.

In noncrosslinked silicone surfactants, a part of methyl group of a linear or branched silicone main chain is substituted with a hydrophilic group such as polyethylene glycol, polyglycerin, etc. Specifically, the component (C) is preferably linear or branched polyoxyethylene-modified organopolysiloxane, linear or branched polyoxyethylene-polyoxypropylene-modified organopolysiloxane, linear or branched polyoxyethylenealkyl-co-modified organopolysiloxane, linear or branched polyoxyethylene-polyoxypropylene-alkyl-co-modified organopolysiloxane, linear or branched polyglycerin-modified organopolysiloxane, linear or branched polyglycerin-alkyl-co-modified organopolysiloxane, or linear or branched pyrrolidone-modified organopolysiloxane. Specific examples thereof include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), Lauryl Polyglyceryl-3 Polydimethylsiloxyethyl Dimethicone (INCI), Bis-Butyldimethicone Polyglyceryl-3 (INCI), etc.

Examples of commercial products thereof include those manufactured by Shin-Etsu Chemical Co.: KF-6011, KF-6011P, KF-6012, KF-6015, KF-6017, KF-6043, KF-6028, KF-6038, KF-6048, KF-6100, KF-6104, KF-6106, KF-6105, KF-6115, etc.

Examples of crosslinked silicone surfactants include crosslinked polyether-modified silicone such as Dimethicone/PEG-10/15 Crosspolymer (INCI), PEG-15/Lauryl Dimethicone Crosspolymer (INCI), PEG-10/Lauryl Dimethicone Crosspolymer (INCI), and PEG-15/Lauryl Polydimethylsiloxyethyl Dimethicone Crosspolymer (INCI); and crosslinked polyglycerin-modified silicone such as Dimethicone/Polyglycerin-3 Crosspolymer (INCI), Lauryl Dimethicone/Polyglycerin-3 Crosspolymer (INCI), Polyglycerin-3/Lauryl Polydimethylsiloxyethyl Dimethicone Crosspolymer (INCI), etc.

When the crosslinked silicone surfactant is used, the crosslinked silicone surfactant is preferably to be swelled, in a composition composed of the crosslinked silicone surfactant and an oil agent being liquid at a room temperature, by containing the liquid oil agent having a weight equal to or larger than that of the crosslinked silicone surfactant.

For the liquid oil agent, liquid silicones, hydrocarbon oils, ester oils, natural animal or vegetable oils, semisynthetic oils, or fluorine-based oils, which may belong to in the components (A) or (B) or (1) oil agent as an optional component described below, can be used. Examples thereof include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), Squalane (INCI), etc.

When the component (A) or (B) is used as a liquid oil agent, the amount of the liquid oil agent shall be included in the content of the component (A) or (B).

Examples of the crosslinked silicone surfactant which is commercially available and swells by incorporating a liquid oil agent include those manufactured by Shin-Etsu Chemical Co.: KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, KSG-850Z, etc.

### [Ultraviolet Absorber]

In the present invention, when a cosmetic is desired to have an ultraviolet shielding effect, it is preferable to include an ultraviolet absorber.

The (A) organosiloxane of the present invention is excellent in compatibility with an organic ultraviolet absorber, and thus cosmetic containing such an organic ultraviolet absorber has a good usability, excellent stability over time and cosmetic durability.

Examples of an ultraviolet absorber include Benzophenone-1 (INCI), Benzophenone-2 (INCI), Benzophenone-3 (INCI), Benzophenone-4 (INCI), Benzophenone-5 (INCI), Benzophenone-6 (INCI), Benzophenone-9 (INCI), Homosalate (INCI), Octocrylene (INCI), Butyl Methoxydibenzoylmethane (INCI), Ethylhexyl Salicylate (INCI), Diethylamino Hydroxybenzoyl Hexyl Benzoate (INCI), Polysilicone-15 (INCI), Ethylhexyl Dimethoxybenzylidene Dioxoimidazolidine Propionate (INCI), Terephthalylidene Dicamphor Sulfonic Acid (INCI), Ethylhexyl Triazone (INCI), Isopentyl Trimethoxycinnamate Trisiloxane (INCI), Drometrizole Trisiloxane (INCI), Ethylhexyl Dimethyl PABA (INCI), Isopropyl Methoxycinnamate (INCI), Ethylhexyl Methoxycinnamate (INCI), Bis-Ethylhexyloxyphenol Methoxyphenyl Triazine (INCI), Phenylbenzimidazole Sulfonic Acid (INCI), Methylene Bis-Benzotriazolyl Tetramethylbutylphenol (INCI), Glyceryl Ethylhexanoate Dimethoxycinnamate (INCI), Glyceryl PABA (INCI), Diisopropyl Methyl Cinnamate (INCI), Cinoxate (INCI), Ethylhexyl Dimethoxybenzylidene Dioxoimidazolidine Propionate (INCI), etc. Further, an UVA absorber (e.g., diethylamino hydroxybenzoyl hexyl benzoate, etc.) and an UVB absorber (e.g., ethylhexyl methoxycinnamate, etc.) can be used together, in any combination thereof.

Among these, one or more kinds of organic ultraviolet absorbers selected from the group consisting of ethylhexyl methoxycinnamate, diethylamino hydroxybenzoyl hexyl benzoate, octyl salicylate, polysilicone-15, t-butyl methoxydibenzoylmethane, oxybenzone, methylene bis-benzotriazolyl tetramethylbutylphenol, bis-ethylhexyloxyphenol methoxyphenyl triazine, and octocrylene, are specifically preferable because these are excellent in compatibility with organosiloxisanes of the present invention.

### [Oil-Based Component in Solid State at 25°C]

In the present invention, if the cosmetic is to be hardened, the cosmetic preferably includes wax, a hydrocarbon, ester, higher alcohol, or higher fatty acid which are solid at 25°C.

Even when an oil-based component which is solid at 25°C is used in combination, the organosiloxane in the inventive cosmetic has high affinity for oil-based components so that the cosmetic can be easily prepared as a stick formulation, etc. without hindering the solidification of the oil-based component. Accordingly, the organosiloxane in the inventive cosmetic is preferable.

The oil-based components being solid at 25°C preferably have a melting point of preferably 40°C or higher, more preferably 60 to 110°C. Such oil-based components can include waxes, hydrocarbons, esters, higher alcohols, and higher fatty acids, and are not particularly limited as long as it is a raw material which can be commonly contained into cosmetics. Specific examples thereof include vegetable waxes such as Copernicia Cerifera (Carnauba) Wax (INCI), saccharum officinarum (sugarcane) wax, Euphorbia Cerifera (Candelilla) Wax (INCI), purified candelilla wax, rice bran wax, japan wax, simmondsia chinensis (jojoba) seed wax, kapok wax, oryza sativa (rice) bran wax, myrica cerifera (bayberry) fruit wax, shea butter, theobroma cacao (cocoa) seed butter, Rhus Succedanea Fruit Wax (INCI), Montan Wax (INCI), and hydrogenated castor oil isostearate; animal waxes such as beeswax, tallow, neat's bone fat, Lard (INCI), Horse Fat (INCI), mutton tallow, Lanolin (INCI), chuhokuro, shellac wax, and spermaceti; semisynthetic oil such as lanolin ester, lanolin fatty acid ester, and beeswax acid ester; hardened oil such as hardened castor oil, and hardened coconut oil; hydrocarbon-based waxes such as solid paraffin, polyethylene, ceresin, ozokerite, and microcrystalline wax; wax ester such as synthetic beeswax; amino acid stearyl alcohol such as dioctyldodecyl lauroyl glutamate, dioctyldodecyl lauroyl glutamate, and dioctyldodecyl lauroyl glutamate; fatty acids such as stearic acid, and behenic acid; and silicone waxes such as acrylic-silicone resins that are acrylic-silicone graft or block copolymers (acrylic-silicone graft copolymers: KP-561P, 562P, manufactured by Shin-Etsu Chemical Co., Ltd.) or derivatives thereof. It is preferable that the oil-based component is one kind or two or more kinds selected from these.

Especially, the oil-based component being solid at 25°C is particularly preferably selected from the group consisting of polyethylene, ceresin, ozokerite, beeswax, microcrystalline wax, stearyl alcohol, behenyl alcohol, and cetanol. This is because the affinity thereof for the organosiloxane (A) in the present invention is particularly high.

### [Other Components]

The inventive cosmetic can further contain a variety of optional components used in general cosmetics, besides the above components (A), (B), and (C), the oil-based components being solid at 25°C, and the ultraviolet absorbers. It may contain, for example, (1) an oil agent, (2) water-based component, (3) a surfactant, (4) a powder, (5) a composition including a crosslinked organopolysiloxane and an oil agent in a liquid state at room temperature, (6) a film-forming agent, (7) an ultraviolet absorbing-scattering agent, (8) another additive. One kind can be used singly, or the mixture of two or more kinds thereof can be used in appropriate combination.

### (1) Oil agent

The oil agent may be volatile or nonvolatile. The examples thereof include nonvolatile silicone oil, natural animal and vegetable oils, semi-synthetic oils, hydrocarbon oils, higher alcohols, fatty acids, ester oils, fluorinated oils, ultraviolet absorbers, etc.

### • Silicone oil

Examples of the silicone oil include alkyl-modified silicones such as Dimeticone (INCI), and Hexyl Dimethicone (INCI); linear or branched organopolysiloxanes having low to high viscosity such as Phenyl Trimethicone (INCI), Diphenyl Dimethicone (INCI), Diphenylsiloxy Phenyl Trimethicone (INCI), Tetraphenyl Dimethyl Disiloxane (INCI), and Methylhydrogenpolysiloxane; amino-modified organopolysiloxanes such as Amodimethicone (INCI), and Aminopropyl Dimethicone (INCI); pyrrolidone-modified organopolysiloxanes such as PCA Dimethicone (INCI); silicone rubber such as pyrrolidone carboxylic acid-modified organopolysiloxane, gum-like dimethylpolysiloxane having a high degree of polymerization, gum-like amino-modified organopolysiloxane, and a gum-like dimethylsiloxane-methylphenylsiloxane copolymer; organopolysiloxane solution of silicone gum or rubber having low viscosity; amino acid-modified silicone; fluorine-modified silicone, silicone resin; a dissolved product of silicone resin, etc.

Examples of commercial products thereof include those manufactured by Shin-Etsu Chemical Co.: KF-96A-6cs, KF-54, KF-54HV, KF-56A, etc.

### • Natural animal and vegetable oil agents and semi-synthetic oil agents

Natural animal and vegetable oil agents and semi-synthetic oil agents include: germ oils such as Persea Gratissima (Avocado) Oil (INCI), Linum Usitatissimum (Linseed) Seed Oil (INCI), Prunus Amygdalus Dulcis (Sweet Almond) Oil (INCI), perilla oil, Olea Europaea (Olive) Fruit Oil (INCI), Torreya Californica (California Nutmeg) Oil (INCI), Cymbopogon Nardus (Citronella) Oil (INCI), Torreya Nucifera Seed Oil (INCI), Kyounin Yu (INCI), Triticum Vulgare (Wheat) Germ Oil (INCI), Sesamum Indicum (Sesame) Seed Oil (INCI), Triticum Vulgare (Wheat) Germ Oil (INCI), Oryza Sativa (Rice) Germ Oil (INCI), Oryza Sativa (Rice) Bran Oil (INCI), Camellia Kissi Seed Oil (INCI), Carthamus Tinctorius (Safflower) Seed Oil (INCI), Glycine Soja(Soybean)Oil (INCI), Camellia Sinensis Seed Oil (INCI), Camellia Japonica Seed Oil (INCI), Oenothera Biennis (Evening Primrose) Oil (INCI), Zea Mays (Corn) Germ Oil (INCI), and Triticum Vulgare (Wheat) Germ Oil (INCI); Natural a vegetable oil such as persic oil, Elaeis Guineensis (Palm) Oil (INCI), Elaeis Guineensis (Palm) Kernel Oil (INCI), Ricinus Communis (Castor) Seed Oil (INCI), Helianthus Annuus (Sunflower) Seed Oil (INCI), Vitis Vinifera (Grape) Seed Oil (INCI), Simmondsia Chinensis (Jojoba) Seed Oil (INCI), Macadamia Ternifolia Seed Oil (INCI), Limnanthes Alba (Meadowfoam) Seed Oil (INCI), Gossypium Herbaceum (Cotton) Seed Oil (INCI), Cocos Nucifera (Coconut) Oil (INCI), and Arachis Hypogaea (Peanut) Oil (INCI); Natural animal oil such as Shark Liver Oil (INCI), Cod Liver Oil (INCI), Fish Liver Oil (INCI), Turtle Oil (INCI), Mink Oil (INCI), and Egg Oil (INCI); semi-synthetic oil and fat such as Hydrogenated Coconut Oil (INCI), and Lanolin Oil (INCI).

### • Hydrocarbon oil

Examples of the hydrocarbon oil include linear or branched hydrocarbon oils. The hydrocarbon oil may be a volatile hydrocarbon oil or a nonvolatile hydrocarbon oil. Specific examples thereof include isoparaffins such as Olefin Oligomer (INCI), and C13-14 Isoparaffin (INCI); alkanes such as Dodecane (INCI), Isohexadecane (INCI), Hydrogenated Polyisobutene (INCI), Squalane (INCI), Mineral Oil (INCI), Coconut Alkanes (INCI), and C13-15 Alkane (INCI).

### • Higher alcohols

Examples of the higher alcohols include linear saturated alcohols having 6 or more carbon atoms such as Lauryl Alcohol (INCI), Hexyldecanol (INCI), Oleyl Alcohol (INCI), Isostearyl Alcohol (INCI), Octyldodecanol (INCI), Decyltetradecanol (INCI), Myristyl Alcohol (INCI), Cetyl Alcohol (INCI), Stearyl Alcohol (INCI), and Behenyl Alcohol (INCI); and Batyl Alcohol (INCI). Further, Examples thereof include sterols such as Cholesterol (INCI), Beta-Sitosterol (INCI), Phytosterols (INCI), Lanosterol (INCI), etc.

### • Ester oils

Examples of the ester oils include: n-alkylglycol monoisostearate such as Diisobutyl Adipate (INCI), dihexyldecyl adipate, Diheptylundecyl Adipate (INCI), and Isostearyl Isostearate (INCI); octyldodecyl esters such as Isocetyl Isostearate (INCI), Trimethylolpropane Triisostearate (INCI), Glycol Diethylhexanoate (INCI), Cetyl Ethylhexanoate (INCI), Trimethylolpropane Triethylhexanoate (INCI), Pentaerythrityl Tetraethylhexanoate (INCI), Cetyl Ethylhexanoate (INCI), and Octyldodecyl Stearoyl Stearate (INCI); palmitic acid ester such as Oleyl Oleate (INCI), Octyldodecyl Oleate (INCI), Decyl Oleate (INCI), Neopentyl Glycol Diethylhexanoate (INCI), Neopentyl Glycol Dicaprate (INCI), Diisostearyl Malate (INCI), Triethyl Citrate (INCI), Diethylhexyl Succinate (INCI), Amyl Acetate (INCI), Etyl Acetate (INCI), Butyl Acetate (INCI), Isocetyl Stearate (INCI), Butyl Stearate (INCI), Diisopropyl Sebacate) (INCI), Diethylhexyl Sebacate (INCI), Cetyl Lactate (INCI), Myristyl Lactate (INCI), Isononyl Isononanoate (INCI), Isotridecyl Isononanoate (INCI), Isopropyl Palmitate (INCI), Ethylhexyl Isopalmitate (INCI), and Isocetyl Palmitate Hexyldecyl Palmitate (INCI); myristic acid esters such as Cholesteryl Hydroxystearate (INCI), Isopropyl Myristate (INCI), Octyldodecyl Myristate (INCI), and Myristyl Myristate (INCI); Ethylhexyl Laurate (INCI); Hexyl Laurate (INCI); Dioctyldodecyl Lauroyl Glutamate (INCI); Isopropyl Lauroyl Sarcosinate (INCI); and Coco-Caprylate·Caprate (INCI), etc.

Among the ester oils, examples of glyceride oil include Triethylhexanoin (INCI), Caprylic/Capric Triglyceride (INCI), Coco-Glycerides (INCI), Caprylic/Capric/Succinic Triglyceride (INCI), Caprylic/Capric Glycerides (INCI), etc.

### • Fluorine-based oil agent

Examples of the fluorine-based oil agent include Perfluorodecalin (INCI), Perfluorononyl Dimethicone (INCI), and Perfluoromethylcyclopentane (INCI), etc.

### (2) Water-based component

Water-based components (water soluble components) are not particularly limited as long as being able to be normally incorporated into cosmetics. Specific examples include water, lower alcohols preferably having 2 to 5 carbon atoms such as Alcohol (INCI) and Isopropyl Alcohol (INCI), sugar alcohols such as Sorbitol (INCI), Maltose (INCI), and Xylitol (INCI). Further, the examples include polyhydric alcohol such as Butylene Glycol (INCI), Propylene Glycol (INCI), Dipropylene Glycol (INCI), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; moisturizing agents such as Glucose (INCI), Glyceryl Glucoside (INCI), Betaine (INCI), Sodium Chondroitin Sulfate (INCI), Sodium PCA (INCI), Methyl Gluceth-10 (INCI), Methyl Gluceth-20 (INCI), hyaluronic acid, egg yolk lecithin, soybean lecithin, phosphatidylcholine, phosphatidylethanolamine, phosphatidyl serine, phosphatidylglycerol, and phosphatidylinositol, sphingophospholipid.

### (3) Surfactant

The surfactant includes nonionic, anionic, cationic, and amphoteric surfactants, but is not particularly limited, and any surfactant can be used as long as it is used for a general cosmetic. In any case, the content of the surfactant is preferably 0.1 to 20% by mass of the whole cosmetic. When the amount is 0.1% or more, functions such as dispersion and emulsification can be sufficiently achieved. When the amount is 20% by mass or less, the cosmetic does not have a sticky touch on use, and thus is preferable. The HLB of the surfactant is not limited and is preferably 2 to 14.5 for the purpose of maintaining the water resistance of the cosmetic.

### (4) Powder

Examples of the powder include color pigment, inorganic powder, metal powder, organic powder, inorganic-organic composite powder, etc. Specific examples thereof are as follows.

### • Color pigment

The color pigment is not particularly limited as long as it is a pigment usually used for colorization of cosmetic. It is possible to use any of red iron oxide (: Labeling Name (INCI: Iron Oxides)), yellow iron oxide (: Labeling Name (INCI: Iron Oxides), white titanium oxide (: Labeling Name (INCI: Titanium Dioxide), black iron oxide (: Labeling Name (INCI: Iron Oxides)), Ultramarines (INCI), iron blue (: Labeling Name (INCI: Ferric Ferrocyanide, Ferric Ammonium Ferrocyanide)), manganese violet (: Labeling Name (INCI: Manganese Violet)), cobalt titanate (: Labeling Name (INCI: Cobalt Titanium Oxide)), chromium hydroxide(: Labeling Name (INCI: Chromium Hydroxide Green)), chromium oxide (: Labeling Name (INCI: Chromium Oxide Greens)), Al/Cobalt oxide (: Labeling Name (INCI: Cobalt Aluminum Oxide)), cobalt titanate (: Labeling Name (INCI: Cobalt Titanium Oxide)), calcined titanium/titanium oxide (: Labeling Name (INCI: Titanium/Titanium Dioxide)), (Li/Cobalt) Titanate (Labeling Name (INCI: Lithium Cobalt Titanate)), cobalt titanate (: Labeling Name (INCI: Cobalt Titanium Oxide)), sintered iron oxide/titanium oxide, composites doped with different metals such as iron oxide-doped titanium oxide (: Labeling Name (INCI: Iron Oxides, Titanium Dioxide)) or the like, an inorganic brown pigment such as Titanium Nitride (: Labeling Name (INCI: Titanium Nitride)), Iron Hydroxide (INCI), γ-iron oxide, or the like, an inorganic yellow pigment such as yellow ocher or the like, colored pigments such as a laked tar-based dye and a laked natural dye, or the like.

Moreover, the pigment may have any shape such as spherical, nearly spherical, rod-like, spindle, petaloid, strip, and irregular forms. The geometrical aspect of the pigment is not particularly limited as long as it can impart color to the cosmetic.

### • Inorganic powder

Examples of the inorganic powder include fine particles made of Zirconium Dioxide (INCI), Zinc Oxide (INCI), Cerium Oxide (INCI), Magnesium Oxide (INCI), Barium Sulfate (INCI), Calcium Sulfate (INCI), Magnesium Sulfate (INCI), Calcium Carbonate (INCI), Magnesium Carbonate (INCI), Talc (INCI), Mica (INCI), Kaolin(INCI), Synthetic Fluorphlogopite (INCI), synthetic phlogopite iron, Biotite (INCI), Potassium Silicate (INCI), Silica (INCI), Aluminum Silicate (INCI), Magnesium Silicate (INCI), Magnesium Aluminum Silicate (INCI), Calcium Silicate (INCI), Aluminum Calcium Sodium Silicate (INCI), Lithium Magnesium Sodium Silicate (INCI) , Sodium Magnesium Silicate (INCI), Calcium Aluminum Borosilicate (INCI), Calcium Sodium Borosilicate (INCI), Hydroxyapatite (INCI), Bentonite (INCI), Montmorillonite (INCI), Hectorite (INCI), zeolite (INCI), Alumina (INCI), Aluminum Hydroxide (INCI), Boron Nitride (INCI), Glass (INCI) or the like.

Moreover, examples of an inorganic color pearl pigment include pearl pigments such as Mica (INCI) coated with Titanium Dioxide (INCI), and Synthetic Fluorphlogopite (INCI) coated with Titanium Dioxide (INCI); Bismuth Oxychloride (INCI), Bismuth Oxychloride (INCI) coated with Titanium Dioxide (INCI), Talc (INCI) coated with Titanium Dioxide (INCI), fish scale guanine (Labeling Name), and colored mica coated with Titanium Dioxide (INCI), etc. The inorganic coloring pigment is not particularly limited to have non-treated surface or surface treated in a known manner used for cosmetics in general.

### • Metal powder

Examples of the metal powder include metal fine particles made of Al (: Labeling Name (INCI: Aluminum, Aluminum Powder)), Copper (: Labeling Name (INCI: Copper Powder)), silver (: Labeling Name (INCI: Silver Powder)), Gold (: Labeling Name (INCI: Gold)), and etc.

### • Organic powder

Examples of the organic powder include powder made of silicone, polyamide, polyacrylic acid-acrylic acid ester, polyester, Polyethylene (INCI), Polypropylene (INCI), Polystyrene (INCI), styrene-acrylic acid copolymer, divinylbenzene-styrene copolymer, polyurethane, vinyl resin, urea resin, melamine resin, benzoguanamine, polymethylbenzoguanamine, tetrafluoroethylene, polymethyl methacrylate, Cellulose (INCI), Silk (INCI), nylon (Labeling Name), phenol resin, epoxy resin, polycarbonate or the like.

In particular, examples of the silicone include silicone resin particles; Polymethylsilsesquioxane (INCI), silicone rubber powder, silicone rubber powder coated with silicone resin; Vinyl Dimethicone/Methicone Silsesquioxane Crosspolymer (INCI), Diphenyl Dimethicone/Vinyl Diphenyl Dimethicone/Silsesquioxane Crosspolymer (INCI), Polysilicone-1 Crosspolymer (INCI), Polysilicone-22 (INCI), etc.

Examples of commercial products of silicone powder include those manufactured by Shin-Etsu Chemical Co.: KMP-590, KMP-591, KMP-592, KMP-597, KMP-598, KSP-100, KSP-101, KSP-102, KSP-105, KSP-300, KSP-411, KSP-441, KM-9729, KM-440, etc.

Further, examples thereof include metal soap and the like. Specific examples thereof include powder made of Zinc Stearate (INCI), Aluminum Stearate (INCI), Calcium Stearate (INCI), Magnesium Stearate (INCI), Zinc Myristate (INCI), Magnesium Myristate (INCI), Sodium Zinc Cetyl Phosphate (INCI), Potassium Cetyl Phosphatezinc Cetyl Phosphate (INCI), etc.

Further, examples of the organic powder include organic dye and the like. Specific examples thereof include tar-based dyes such as Red No. 3, Red No. 104 (1) (: Labeling name (INCI: Red 28, Red 28 Lake)), Red No. 106, Red No. 201 (: Labeling name (INCI: Red 6)), Red No. 202 (: Labeling name (INCI: Red 7)), Red No. 204, Red No. 205, Red No. 220 (: Labeling name (INCI: Red 34)), Red No. 226 (: Labeling name (INCI: Red 30)), Red No. 227 (: Labeling name (INCI: Red 33, RED 33 Lake)), Red No. 228 (: Labeling name (INCI: Red 36)), Red No. 230 (1) (: Labeling name (INCI: Red 22, Red 22 Lake)), Red No. 230 (2) (Labeling Name), Red No. 401 (Labeling Name), Red No. 505 (Labeling Name), Yellow No. 4 (: Labeling name (INCI: Yellow 5)), Yellow No. 5 (: Labeling name (INCI: Yellow 6, Yellow 6 Lake)), Yellow No. 202 (1) (: Labeling name (INCI: Yellow 8)), Yellow No. 203 (: Labeling name (INCI: Yellow 10, Yellow 10 Lake)), Yellow No. 204 (: Labeling name (INCI: Yellow 11)), Yellow No. 401, Blue No. 1 (: Labeling name (INCI: Blue 1, Blue 1 Lake)), Blue No. 2, Blue No. 201, Blue No. 205 (: Labeling name (INCI: Blue 4)), Blue No. 404 (Labeling Name), Green No. 3 (: Labeling name (INCI: Green 3, Green 3 Lake)), Green No. 201 (: Labeling name (INCI: Green 5)), Green 202 (: Labeling name (: Green 6)), Green No. 204 (: Labeling name (INCI: Green 8)), Green No. 205 (Labeling name), Orange No. 201 (:Labeling name (INCI: Orange 5)), Orange No. 203 (:Labeling name (INCI: Pigment Orange 5)), Orange No. 204 (Labeling Name), Orange No. 205 (: Labeling Name (INCI: Orange 4, Orange 4 Lake)), Orange No. 206 (: Labeling Name (INCI: Orange 10)), Orange No. 207 (Labeling Name (INCI: Orange 11)), etc.; and natural dyes such as Cochineal (INCI), Laccaic Acid (INCI), Carthamus Tinctorius (Safflower) Flower Extract (INCI), Lithospermum Officinale Root Extract (INCI), Lithospermum Erythrorhizon Root Extract (INCI), gardenia yellow pigment (Labeling Name), Hydrolyzed Gardenia Florida Extract (INCI), etc.

### • Inorganic-organic composite powder

Examples of the inorganic-organic composite powder include composite powder in which the surface of inorganic powder is coated with organic powder by a publicly-known general method.

Note that the above-described powders can be used also after treating their surface. From the viewpoint of water resistance of the cosmetic, the surface treatment agent is preferably capable of imparting hydrophobicity, and such a surface treatment agent are not particularly limited. Examples thereof include silicone treatment agents; waxes; paraffins; organofluorine compounds of perfluoroalkyl and phosphate or the like; a surfactant; and an amino acid such as N-acyl glutamic acid; a metal soap such as aluminum stearate, and magnesium myristate.

The silicone treatment agent is more preferable, and examples thereof include silanes such as Triethoxycaprylylsilane (INCI) or silylating agent, Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethyl Hexyl Dimethicone (ICI), Acrylates/Tridecyl Acrylate/Triethoxysilylpropyl Methacrylate/Dimethicone Methacrylate Copolyme (INCI), etc.

Specific examples of the silicone treatment agent include those manufactured by Shin-Etsu Chemical Co. Ltd.: AES-3083, KF-99P, KF-9901, KF-9908, KF-9909, KP-574, KP-541, etc.

Further, one of these surface-hydrophobizing treatment agents may be used alone or two or more thereof may be used in combination. Specific examples of a surface-treated color pigment include KTP-09 series manufactured by Shin-Etsu Chemical Co., Ltd., especially KTP-09W, 09R, 09Y, 09B, etc.

### (5) Composition including crosslinked organopolysiloxane and oil agent in liquid state at room temperature

In the composition including a crosslinked organopolysiloxane and an oil agent in a liquid state at room temperature, the crosslinked organopolysiloxane is preferably to be swelled by incorporating the liquid oil in an amount of equal to or more than the weight of the crosslinked organopolysiloxane. The liquid oil agent to be used can be liquid silicone oil, hydrocarbon oil, ester oil, natural animal or vegetable oil, semisynthetic oil, or fluorine-based oil belonging to the oil agent (1) as the optional component. Examples thereof include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), Squalane (INCI), etc.

Different from a crosslinked silicone surfactant of the component (3) described above, the component (5) is a compound having no polyether or polyglycerol structure in the molecular structure, and specific examples thereof include Dimethicone/Vinyl Dimethicone Crosspolymer (INCI), Dimethicone/Phenyl Vinyl Dimethicone Crosspolymer (INCI), Vinyl Dimethicone/Lauryl Dimethicone Crosspolymer (INCI), Lauryl Polydimethylsiloxyethyl Dimethicone/Bis-Vinyldimethicone Crosspolymer (INCI), etc.

Example of commercial products of the composition including a crosslinked organopolysiloxane and an oil agent in a liquid state at room temperature include those manufactured by Shin-Etsu Chemical Co., Ltd.: KSG-15, KSG-1510, KSG-16, KSG-1610, KSG-19, KSG-016F, KSG-18A, KSG-41A, KSG-42A, KSG-43, KSG-44, KSG-042Z, KSG-045Z, KSG-048Z, etc.

### (6) Film-forming agent

The film-forming agent is contained mainly to further maintain the durability of the effect of the cosmetic. The film-forming agent is not particularly limited but is preferably a silicone-based composition from the viewpoint of imparting water repellency. Specifically, it is possible to use trimethylsiloxysilicate, acrylic-silicone film-forming agent, silicone-modified norbornene, silicone-modified pullulan, silicone-modified polyvinyl alcohol, or the like.

Examples of the film-forming agent of the silicone-based composition include Trimethylsiloxysilicate (INCI), Acrylates/Dimethicone Copolymer (INCI), Norbornene/Tris(Trimethylsiloxy) Silylnorbornene Copolymer (INCI), Trimethylsiloxysilylcarbamoyl Pullulan (INCI), etc.

The film-forming agent may be contained in the cosmetic after being dissolved in an oil agent in a liquid state at room temperature. For the liquid oil agent, it is possible to use silicone oil, hydrocarbon oil, ester oil, natural animal or vegetable oil, semisynthetic oil, or fluorine-based oil belonging to (1) oil agent as the optional component.

Example of the commercial product of silicon film-forming agents include those manufactured by Shin-Etsu Chemical Co., Ltd.: KF-7312J, KP-545, KP-549, KP-543, NBN-30-ID, TSPL-30-ID, TSPL-30-D5, etc.

### (7) Ultraviolet absorbing-scattering agent

Examples of the ultraviolet absorbing-scattering agent include particles that absorb and/or scatter ultraviolet light, such as titanium oxide fine particles, iron-containing titanium oxide fine particles, zinc oxide fine particles, cerium oxide fine particles, and composites thereof. It is also possible to use dispersion in which these particles that absorb and/or scatter ultraviolet light are dispersed in an oil agent in advance.

For the oil agent, it is possible to use liquid silicone oil, hydrocarbon oil, ester oil, natural animal or vegetable oil, semisynthetic oil, or fluorine-based oil.

Specific examples of the dispersion in which particles that absorb and/or scatter ultraviolet light are dispersed in an oil agent in advance include SPD series (product name) manufactured by Shin-Etsu Chemical Co., Ltd., particularly SPD-T5, T5L, Z5, Z5L, T6, Z6, T7, etc.

### (8) Other Additives

Examples of the other additive include an oil-soluble gellant, a preservative·microbicide, an antiperspirant, a fragrance, a salt, an antioxidant, a pH adjuster, a chelator, a refrigerant, an anti-inflammatory agent, a component for skin care (a skin-brightening agent, a cell activator, a rough skin-improving agent, a blood circulation promoter, a skin astringent, an antiseborrheic agent, etc.), vitamins, amino acids, nucleic acids, hormone, an inclusion compound, etc.

### • Oil-soluble gellant

Examples of the oil-soluble gellant include: metal soap such as aluminum stearate, magnesium stearate, and zinc myristate; amino acid derivatives such as Lauroyl Glutamic Acid (INCI), and α,γ-di-n-butylamine; dextrin fatty acid esters such as Dextrin Palmitate (INCI), Dextrin Isostearate (INCI), Dextrin Myristate (INCI), Stearoyl Inulin (INCI), and Dextrin Palmitate/Ethylhexanoate (INCI); sucrose fatty acid esters such as sucrose palmitic acid ester, and sucrose stearic acid ester; fructooligosaccharide fatty acid ester such as fructooligosaccharide stearate ester, and fructooligosaccharide 2-ethylhexanoate; benzylidene derivative of sorbitol such as monobenzylidene sorbitol, and dibenzylidene sorbitol; organically modified clay mineral such as Disteardimonium Hectorite (INCI), Stearalkonium Hectorite (INCI), Organic Denaturing hectorite clay; and Stearalkonium Bentonite (INCI).

### • Preservative·Microbicide

Examples of the preservative·microbicide include p-hydroxybenzoic acid alkyl ester, benzoic acid, sodium benzoate, sorbic acid, potassium sorbate, phenoxyethanol, imidazolidinylurea, salicylic acid, isopropyl methylphenol, carbolic acid, para chloro meta cresol, hexachlorophene, benzalkonium chloride, chlorhexidine chloride, trichlorocarbanilide, iodopropynyl butyl carbamate, polylysine, photosensitizer, silver, and plant extract.

### • Antiperspirant

Examples of the antiperspirant include aluminum hydroxyhalide such as chlorohydroxy aluminum; aluminum halide such as aluminum chloride; aluminum allantoinate, tannic acid, persimmon tannin, potassium aluminum sulfate, zinc oxide, zinc para-phenolsulfonate, burnt alum, aluminum zirconium tetrachlorohydrate, aluminum zirconium trichlorohydrex glycine, etc. In particular, as a component exhibiting a high effect, the preferable antiperspirants are aluminum hydroxyhalide, aluminum halide, and a complex or mixture thereof with zirconyl oxyhalide and zirconyl hydroxyhalide, which is such as aluminum zirconium tetrachlorohydrate, and aluminum zirconium trichlorohydrex glycine.

### • Fragrance

The fragrance includes natural fragrance and synthetic fragrance. Examples of natural fragrances include vegetable fragrance separated from a flower, a leaf, wood, a peel, etc.; and animal fragrance such as musk and civet. Examples of the synthetic fragrance include hydrocarbons such as monoterpene,; alcohols such as aliphatic alcohol and aromatic alcohol; aldehydes such as terpene aldehyde and aromatic aldehyde; ketones such as alicyclic ketone; esters such as terpene-based ester; lactones; phenols; oxides; nitrogen-containing compounds; acetals; etc.

### • Salt

Examples of the salts include an inorganic salt, an organic acid salt, an amine salt, and an amino acid salt. Examples of the inorganic salt include a sodium salt, a potassium salt, a magnesium salt, a calcium salt, an aluminum salt, a zirconium salt, a zinc salt, etc. of inorganic acids such as hydrochloric acid, sulfuric acid, carbonic acid, and nitric acid. Examples of the organic acid salt include a salt of organic acid such as acetic acid, dehydroacetic acid, citric acid, malic acid, succinic acid, ascorbic acid, and stearic acid. Examples of the amine salt and the amino acid salt include a salt of amine such as triethanolamine; and a salt of amino acid such as glutamic acid. Besides, it is also possible to use a salt of hyaluronic acid, chondroitin sulfate, etc.; aluminum zirconium glycine complex, etc.; an acid-alkali neutralization salt used in preparation of the cosmetics; or the like.

### • Antioxidant

Examples of the antioxidant include, but are not particularly limited to, carotenoid, ascorbic acid and salt thereof, ascorbyl stearate, tocophenol, tocophenol acetate, tocopherol, p-t-butylphenol, butylhydroxyanisol, dibutylhydroxytoluene, phytic acid, ferulic acid, thiotaurine, hypotaurine, sulfite, erythorbic acid and salt thereof, chlorogenic acid, epicatechin, epigallocatechin, epigallocatechin gallate, apigenin, campherol, myricetin, quercetin, etc. One of the antioxidants may be used alone, or two or more thereof may be used in combination.

### · pH adjuster

Examples of the pH adjuster include lactic acid, citric acid, glycolic acid, succinic acid, tartaric acid, dl-malic acid, potassium carbonate, sodium bicarbonate, ammonium bicarbonate, etc.

### • Chelator

Examples of the chelator include alanine, sodium edetate, sodium polyphosphate, sodium metaphosphate, phosphoric acid, etc.

### • Cooling agent

Examples of the cooling agent include L-menthol, camphor, menthyl lactate, etc.

### • Anti-inflammatory agent

Examples of the anti-inflammatory agent include allantoin, glycyrrhizinic acid and salt thereof, glycyrrhetinic acid and stearyl glycyrrhetinate, tranexamic acid, azulene, etc.

### • Component for skin care

Examples of the components for skin care include: skin-brightening agents such as placenta extract, arbutin, glutathione, and strawberry geranium extract; cell activators such as royal jelly, photosensitizer, cholesterol derivative, and calf blood extract; rough skin-improving agents; blood circulation promoters such as vanillylamide nonylate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-orizanol; skin astringents such as zinc oxide and tannic acid; antiseborrheic agents such as sulfur thianthrol.

### • Vitamin

Examples of the vitamins include vitamin A such as vitamin A oil, retinol, retinol acetate, and retinol palmitate; vitamin B2 such as riboflavin, riboflavin butyrate, and flavin adenine nucleotide; vitamin B6 such as pyridoxine hydrochloride, pyridoxine dioctanoate, and pyridoxine tripalmitate; vitamin B such as vitamin B12 and derivative thereof, and vitamin B15 and derivative thereof; vitamin C such as L-ascorbic acid, L-ascorbic acid dipalmitate, sodium L-ascorbic acid-2-sulfate, and L-ascorbic acid diester dipotassium phosphate dipotassium phosphate; vitamin D such as ergocalciferol, and cholecalciferol; vitamin E such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and amide nicotinate; vitamin H; vitamin P; pantothenic acids such as calcium pantothenate, D-pantothenyl alcohol, pantothenyl ethyl ether, and acetyl pantothenyl ethyl ether; biotin; etc.

### • Amino acid

Examples of the amino acids include glycine, valine, leucine, isoleucine, serine, threonine, phenylalanine, arginine, lysine, aspartic acid, glutamic acid, cystine, cysteine, methionine, tryptophan, etc.

### • Nucleic acid

Examples of the nucleic acids include deoxyribonucleic acid, etc.

### • Hormone

Examples of the hormone include estradiol, ethenyl estradiol, etc.

### • Inclusion compound

Examples of the inclusion compounds include cyclodextrin, etc.

### [Forms and Application of Cosmetics]

Examples of the form of the inventive cosmetic include powder, oil-based liquid, water-in-oil emulsions, oil-in-water emulsions, non-aqueous emulsions, multi-emulsions such as W/O/W and O/W/O. Further, the forms of the inventive cosmetics can be selected from a variety of forms such as liquid, emulsion, cream, solid, paste, gel, powder, pressed, multilayer, mousse, spray, stick, and pencil.

The specific embodiment of the inventive cosmetic is not particularly limited as long as containing the essential components, but can be applied for such as skin care cosmetics, liquid foundation, powder foundation, concealer, lipstick, a variety of products in which sunscreen function imparted, etc.

### EXAMPLES

Hereinafter, the present invention will be specifically described with reference to Examples and Comparative Examples, but the present invention is not limited thereto. Note that in Tables shown below, "Ex." means "Example" and "Com. Ex." means "Comparative Example".

### [Production of Component (A)]

According to the following Production Example 1, organosiloxane Sx used for as the component (A) in the inventive cosmetic were produced.

### (Production Example 1)

### 3,5-diethyl-1,1,1,3,5,7,7,7-octamethyltetrasiloxane

A 1-L four-necked flask was charged with 282.6 g of 1,1,1,3,5,7,7,7-octamethyltetrasiloxane and 1.4 mg of chloroplatinic acid. Ethylene gas was blown into the mixture under stirring, and a hydrosilylation reaction took place while the temperature was held at 60 to 70°C. The reaction was monitored by gas chromatography. When the proportion of the target product reached 95% or more, the reaction was terminated. Distillation under reduced pressure (120°C, 10 mmHg) was performed to obtain 3,5-diethyl-1,1,1,3,5,7,7,7-octamethyltetrasiloxane as the target Sx. The boiling point was 227°C and the kinematic viscosity at 25°C was 2.2 mm²/s.

Hereinafter, formulation examples of cosmetics in Examples and Comparative Examples will be described. In the below, blended amounts are expressed in % (% by mass) unless otherwise specified.

### (Examples 1 to 4, Comparative Examples 1 to 5) W/O type Foundation

W/O type foundations were prepared according to the compounding ratio shown in Table 1 below.

**[Table 1]**

| | | Ex. 1 | Ex. 2 | Ex. 3 | Ex. 4 | Com. Ex. 1 | Com. Ex. 2 | Com. Ex. 3 | Com. Ex. 4 | Com. Ex. 5 |
|---|---|---|---|---|---|---|---|---|---|---|
| 1 | Sx of Production Ex. 1 | 8.0 | 8.0 | 29.5 | 8.0 | 37.5 | - | - | - | - |
| 2 | TMF-1.5 (Note 1) | 29.5 | - | - | - | - | 37.5 | - | - | - |
| 3 | Dimethicone 1.5CS | - | 29.5 | 8.0 | 21.5 | - | - | 37.5 | - | - |
| 4 | Dimethicone 2CS | - | - | - | 8.0 | - | - | - | 37.5 | - |
| 5 | Clopentasiloxane | - | - | - | - | - | - | - | - | 37.5 |
| 6 | Ethylhexyl Methoxycinnamate | 7.5 | 7.5 | 7.5 | 7.5 | 7.5 | 7.5 | 7.5 | 7.5 | 7.5 |
| 7 | KSG-210 (Note 2) | 3.5 | 3.5 | 3.5 | 3.5 | 3.5 | 3.5 | 3.5 | 3.5 | 3.5 |
| 8 | KSG-15 (Note 3) | 5.0 | 5.0 | 5.0 | 5.0 | 5.0 | 5.0 | 5.0 | 5.0 | 5.0 |
| 9 | KF-6028 (Note 4) | 2.0 | 2.0 | 2.0 | 2.0 | 2.0 | 2.0 | 2.0 | 2.0 | 2.0 |
| 10 | Disteardimonium Hectorite | 1.2 | 1.2 | 1.2 | 1.2 | 1.2 | 1.2 | 1.2 | 1.2 | 1.2 |
| 11 | KP-578 (Note 5) | 0.3 | 0.3 | 0.3 | 0.3 | 0.3 | 0.3 | 0.3 | 0.3 | 0.3 |
| 12 | KTP-09W (Note 6) | 8.0 | 8.0 | 8.0 | 8.0 | 8.0 | 8.0 | 8.0 | 8.0 | 8.0 |
| 13 | KTP-09R (Note 6) | 1.6 | 1.6 | 1.6 | 1.6 | 1.6 | 1.6 | 1.6 | 1.6 | 1.6 |
| 14 | KTP-09Y (Note 6) | 0.7 | 0.7 | 0.7 | 0.7 | 0.7 | 0.7 | 0.7 | 0.7 | 0.7 |
| 15 | KTP-09B (Note 6) | 0.2 | 0.2 | 0.2 | 0.2 | 0.2 | 0.2 | 0.2 | 0.2 | 0.2 |
| 16 | Dipropylene glycol | 5.0 | 5.0 | 5.0 | 5.0 | 5.0 | 5.0 | 5.0 | 5.0 | 5.0 |
| 17 | Methylparaben | 0.3 | 0.3 | 0.3 | 0.3 | 0.3 | 0.3 | 0.3 | 0.3 | 0.3 |
| 18 | Sodium Citrate | 0.2 | 0.2 | 0.2 | 0.2 | 0.2 | 0.2 | 0.2 | 0.2 | 0.2 |
| 19 | Sodium Chloride | 0.5 | 0.5 | 0.5 | 0.5 | 0.5 | 0.5 | 0.5 | 0.5 | 0.5 |
| 20 | Ethanol | 3.0 | 3.0 | 3.0 | 3.0 | 3.0 | 3.0 | 3.0 | 3.0 | 3.0 |
| 21 | Water | 23.5 | 23.5 | 23.5 | 23.5 | 23.5 | 23.5 | 23.5 | 23.5 | 23.5 |
| | Total | 100.0 | 100.0 | 100.0 | 100.0 | 100.0 | 100.0 | 100.0 | 100.0 | 100.0 |

| | | | | | | | | | | |
|---|---|---|---|---|---|---|---|---|---|---|
| (Note 1) manufactured by Shin-Etsu Chemical Co., Ltd.: methyl trimethicone (Note 2) manufactured by Shin-Etsu Chemical Co., Ltd.: mixture of 70-80% dimethicone 6cs and 20-30% (dimethicone/(PEG-10/15)) crosspolymer (Note 3) manufactured by Shin-Etsu Chemical Co., Ltd.: Mixture of 90-96% cyclopentasiloxane and 4-10% (dimethicone/vinyl dimethicone) crosspolymer (Note 4) manufactured by Shin-Etsu Chemical Co., Ltd.: PEG-9 polydimethylsiloxyethyl dimethicone (Note 5) manufactured by Shin-Etsu Chemical Co., Ltd.: (acrylates/ethylhexyl acrylate/dimethicone methacrylate) copolymer (Note 6) manufactured by Shin-Etsu Chemical Co., Ltd.: KF-9909-treated color inorganic pigment, W: white, R: red, Y: yellow, B: black | | | | | | | | | | |

TMF-1.5 was volatile oil, and the kinematic viscosity measured according to the method described above at 25°C was 1.5 mm²/s. Dimethicone 1.5CS was volatile oil, and the kinematic viscosity measured according to the method described above at 25°C was 1.5 mm²/s. Dimethicone 2CS is volatile oil, and the kinematic viscosity measured according to the method described above at 25°C was 2 mm²/s. That is, TMF-1.5, Dimethicone 1.5CS, and Dimethicone 2CS are the component (B) of the present invention.

### (Production Method)

Portions of Components 1 to 5 and Components 6 to 10 according to the compounding ratio shown in Table 1, were stirred and uniformly mixed. To the mixture, a mixture of Components 16 to 21 prepared separately by uniformly dissolving Components 16 to 20 in Component 21 were quietly added and stirred to obtain an emulsified product. Separately, the remainder of Components 1 to 5, and Components 11 to 15 according to the compounding ratio shown in Table 1, were treated with a roller, added to the emulsified product, and mixed. The resultant was placed in a predetermined container. In this manner, W/O type foundations were obtained.

For the obtained W/O type foundations, a use test was performed by ten female expert panels to evaluate (1) feeling (light feeling or not), (2) film uniformity, and (3) durability of cosmetic, according to the following evaluation criteria. Moreover, (4) a state of the cosmetic after being left to stand at 40°C for one month was also observed. Table 2 shows the evaluation results of Examples 1 to 4 and Comparative Examples 1 to 5.

### (Evaluation Criteria)

Each expert panel graded evaluation items according to the below.
5 points: very good
4 points: good
3 points: fair
2 points: slightly poor
1 point: poor
The graded scores were averaged and was judged according to the following criteria.

### Judgement of Average:

The obtained average is 4.5 points or more: A
The obtained average is 3.5 points or more and less than 4.5 points: B
The obtained average is 2.5 points or more and less than 3.5 points: C
The obtained average is 1.5 points or more and less than 2.5 points: D
The obtained average is less than 1.5 points: E

**[Table 2]**

| No. | Evaluation item | Ex.1 | Ex.2 | Ex.3 | Ex.4 | Com. Ex. 1 | Com. Ex. 2 | Com. Ex. 3 | Com. Ex. 4 | Com. Ex. 5 |
|---|---|---|---|---|---|---|---|---|---|---|
| (1) | Feeling | B | B | A | B | B | B | B | B | B |
| (2) | Film Uniformity | A | A | A | A | A | C | C | C | A |
| (3) | Durability of Cosmetic | A | A | A | A | B | C | C | C | B |
| (4) | State of cosmetic | Stable | Stable | Stable | Stable | Stable | Separated | Separated | Separated | Separated |

As clearly shown in Table 2, W/O foundations of Examples 1 to 4, which contain both the components (A) and (B) explained before, were verified to have a good feeling, good film uniformity, and good cosmetic durability. Additionally, W/O foundations of Examples 1 to 4 are revealed to be excellent in storage stability. Note that, W/O foundations of Examples 1 to 4 had an excellent spread, although it is not shown in Table 2.

On the other hand, as for W/O foundation of Comparative Example 1, which did not contain the component (B), all expert panels answered that it had poorer cosmetic durability than those of W/O foundations of Examples 1 to 4

As shown in Table 2, W/O foundation of Comparative Examples 2 to 5, which did not contain the component (A), separated after being left to stand at 40°C for one month. Furthermore, as for W/O foundation of Comparative Examples 2 to 4, all expert panels answered that it had poor film uniformity and poor cosmetic durability.

### (Examples 5) Sunscreen Emulsion (Shaking)

In accordance with the compounding ratio shown in the following Table 3 and production method explained in the below, a sunscreen emulsion (shaking) of Example 5 was produced.

**[Table 3]**

| Component | | Mass (%) |
|---|---|---|
| 1. Dimethicone1.5CS (Component (B)) | | 17.5 |
| 2. Organosiloxane obtained in Production Example 1 (Component (A)) | | 9 |
| 3. KF-56A (Note 1) | | 5 |
| 4. KSG-18A (Note 2) | | 3 |
| 5. KF-6038 (Note 3) | | 2 |
| 6. Ethylhexyl Methoxycinnamate | | 7.5 |
| 7. Diethylamino Hydroxybenzoyl Hexyl Benzoate | | 1 |
| 8 .Octocrylene | | 2.5 |
| 9. Disteardimonium Hectorite | | 1 |
| 10. KP-545 (Note 4) | | 2 |
| 11. KSP-1 05 (Note 5) | | 0.5 |
| 12. SPD-T5 (Note 6) | | 5 |
| 13. SPD-Z5 (Note 7) | | 10 |
| 14. BG | | 3 |
| 15. Sodium Citrate | | 0.2 |
| 16. Sodium Chloride | | 0.5 |
| 17. Ethanol | | 5 |
| 18. Water | | 25.3 |
| | Total | 100 |

| | | |
|---|---|---|
| (Note 1) manufactured by Shin-Etsu Chemical Co., Ltd.: diphenylsiloxy phenyl trimethicone (Note 2) manufactured by Shin-Etsu Chemical Co., Ltd.: mixture of 80-90% diphenylsiloxy phenyl trimethicone and 10-20% dimethicone/phenyl vinyl dimethicone crosspolymer (Note 3) manufactured by Shin-Etsu Chemical Co., Ltd.: lauryl PEG-9 polydimethylsiloxyethyl dimethicone (Note 4) manufactured by Shin-Etsu Chemical Co., Ltd.: dissolved product of 70% cyclopentasiloxane and 30% acrylates/dimethicone copolymer (Note 5) manufactured by Shin-Etsu Chemical Co., Ltd.: vinyl dimethicone/methicone silsesquioxane crosspolymer (Note 6) manufactured by Shin-Etsu Chemical Co., Ltd.: cyclopentasiloxane dispersion of 40% titanium oxide fine particles (Note 7) manufactured by Shin-Etsu Chemical Co., Ltd.: cyclopentasiloxane dispersion of 60% zinc oxide fine particles | | |

Dimethicone 1.5CS as the Component 1 was volatile oil, and the kinematic viscosity measured according to the method described above at 25°C was 1.5 mm²/s.

### (Production Method)

A: Components 1 to 10 were uniformly mixed (Mixture A).
B: Component 11 was added to Mixture A and uniformly dispersed (Dispersion B).
C: Components 14 to 17 were added to Component 18 and dissolved (Solution C).
D: Solution C was gradually added to Dispersion B and emulsified, and Components 12 and 13 were added to obtain sunscreen emulsion.

It was found that the sunscreen cream obtained as described above had light spreadability, a dry felling without stickiness, no change due to temperature or over time, and was very excellent in usability and stability.

### (Example 6) Sunscreen Cream

In accordance with the compounding ratio shown in the following Table 4 and production method explained in the below, sunscreen cream of Example 6 was produced.

**[Table 4]**

| Component | Mass (%) |
|---|---|
| 1. Organosiloxane obtained in Production Example 1(Component (A)) | 20 |
| 2. Light isoparaffin (Component (B)) | 10 |
| 3. Squalane | 3 |
| 4. KF-6105 (Note 1) | 4 |
| 5. Ethylhexyl Methoxycinnamate | 7.5 |
| 6. t-Butyl Methoxydibenzoylmethane | 3 |
| 7. Polysilicone-15 | 1 |
| 8. Distearyldimonium Chloride | 1 |
| 9. Tocophenol Acetate | 0.1 |
| 10. Ethanol | 1 |
| 11. Sodium Citrate | 0.5 |
| 12. Magnesium Sulfate | 0.5 |
| 13. Preservative | 0.3 |
| 14. Water | 48.1 |
| Total | 100 |

| | |
|---|---|
| (Note 1) manufactured by Shin-Etsu Chemical Co., Ltd.: lauryl polyglyceryl-3 polydimethylsiloxyethyl dimethicone | |

Light isoparaffin of Component 2 was volatile oil, and the kinematic viscosity measured according to the method described above at 25°C was 1.8 mm²/s.

### (Production Method)

A: Components 1 to 9 were uniformly mixed (Mixture A).
B: Components 10 to 13 were uniformly dissolved in Component 14 (Solution B).
C: Solution B was gradually added to Mixture A under stirring, and emulsified to obtain a sunscreen cream.

It was found that the sunscreen cream obtained as described above had fine texture and light spreadability, gave a wet and fresh feeling, did not attach sand at all due to the absence of stickiness, and had quite favorable usability. Further, it was found that the sunscreen cream did not change due to temperature or over time and had excellent stability.

### (Example 7) Lipstick

In accordance with the compounding ratio shown in the following Table 5 and production method explained in the below, lipstick of Example 7 was produced.

**[Table 5]**

| Component | Mass (%) |
|---|---|
| 1. Organosiloxane obtained in Production Example 1 (Component (A)) | 18.8 |
| 2. Dimethicone1.5CS (Component (B)) | 10 |
| 3. Isotridecyl Isononanoate | 10 |
| 4. Diisostearyl Malate | 5 |
| 5. Candelilla wax | 10 |
| 6. Polyethylene | 7 |
| 7. Microcrystalline wax | 2 |
| 8. KF-7312L (Note 1) | 20 |
| 9. KSG-330 (Note 2) | 5 |
| 10. KF-6105 (Note 3) | 3 |
| 11. Colorant | 1.2 |
| 12. Mica | 8 |
| Total | 100 |

| | |
|---|---|
| (Note 1) manufactured by Shin-Etsu Chemical Co., Ltd.: dissolved product of 50% Trimethylsiloxysilicate in dimethicone2CS (Note 2) manufactured by Shin-Etsu Chemical Co., Ltd.: mixture of 65-75% triethylhexanoin and 25-35% alkyl branched, crosslinked dimethylpolysiloxane (Note 3) manufactured by Shin-Etsu Chemical Co., Ltd.: lauryl polyglyceryl-3 polydimethylsiloxyethyl dimethicone | |

Dimethicone 1.5CS as the Component 2 was volatile oil, and the kinematic viscosity measured according to the method described above at 25°C was 1.5 mm²/s.

### (Production Method)

A: Component 11 was mixed with a portion of Component 3 and dispersed using a roller mill, followed by heating and mixing the obtained dispersion, Components 1 and 2, the remainder of Component 3, Components 4 to 10, and Components 12 (Mixture A) .
B: Mixture A was poured into a mold, cooled, and solidified, to obtain a lipstick of stick-shaped Example 7 .

The lipstick obtained as described above had good spreadability, good film uniformity, and cosmetic sustainability. Further, it was revealed that the obtained lipstick was excellent in storage stability.

### (Example 8) Oil-based Solid Foundation

In accordance with the compounding ratio shown in the following Table 6 shown below and production method explained in the below, oil-based solid foundation of Example 8 was produced.

**[Table 6]**

| Component | | | Mass (%) |
|---|---|---|---|
| | 1. Synthetic wax | | 4.0 |
| | 2. Carnauba wax | | 2.0 |
| | 3. Shea fat | | 0.5 |
| | 4. Caprylic/capric triglyceride | | 3.0 |
| | 5. KF-56A (Note 1) | | 5.0 |
| | 6. Ethylhexyl methoxycinnamate | | 7.0 |
| | 7. Bis-ethylhexyloxyphenol methoxyphenyl triazine | | 0.5 |
| | 8. Organosiloxane obtained in Production Example 1 (Component(A)) | | 4.0 |
| | 9. Dodecane (Component (B)) | | 1.0 |
| | 10. Isotridecyl isononanoate | | residual amount |
| | 11. KSP-100(Note 2) | | 4.0 |
| | 12. KMP-591(Note 3) | | 1.0 |
| | 13. Cetyl ethylhexanoate | | 3.0 |
| | 14. KF-6115(Note 4) | | 1.0 |
| | 15. Titanium oxide fine particles treated by KF-99P (Note 5) | | 7.0 |
| | 16. KTP-09W (Note 6) | | appropriate amount |
| | 17. KTP-09R (Note 6) | | appropriate amount |
| | 18. KTP-09Y (Note 6) | | appropriate amount |
| | 19. KTP-09B (Note 6) | | appropriate amount |
| | | Total | 100.0 |

| | | | |
|---|---|---|---|
| (Note 1) manufactured by Shin-Etsu Chemical Co., Ltd.: diphenylsiloxy phenyl trimethicone (Note 2) manufactured by Shin-Etsu Chemical Co., Ltd.: (vinyl dimethicone/methicone silsesquioxane) crosspolymer (Note 3) manufactured by Shin-Etsu Chemical Co., Ltd.: polymethylsilsesquioxane (Note 4) manufactured by Shin-Etsu Chemical Co., Ltd.: lauryl polyglyceryl-3 polydimethylsiloxyethyl dimethicone (Note 5) manufactured by Shin-Etsu Chemical Co., Ltd.: methicone-treated (Note 6) manufactured by Shin-Etsu Chemical Co., Ltd.: triethoxysilylethyl polydimethylsiloxyethyl hexyl dimethicone-treated color inorganic pigment, W: white, R: red, Y: yellow, B: black | | | |

Dodecane as the Component 9 was volatile oil, and the kinematic viscosity measured according to the method described above at 25°C was 1.85 mm²/s.

### (Production Method)

A: Components 1 to 10 were heated and dissolved.
B: Components 13 to 19 were uniformly mixed and then subjected to a roll treatment.
C: The product obtained in the above process B and the components 11 to 12 were added to the product obtained in the above heating process A, and the mixture was uniformly mixed.
D: The product obtained by the above process C was defoamed, placed in a container under heating, and cooled down to a room temperature, to obtain oil-based solid foundation of Example 8.

The inventive oil-based solid foundation obtained as described above had light spreadability and good fitting to the skin, gave a moist finish, suppressed shine, provided a firm cosmetic film, had good cosmetic durability, and was extremely excellent.

### (Example 9) Oil-based Foundation

In accordance with the compounding ratio shown in the following Table 7 shown below and production method explained in the below, oil-based foundation of Example 9 was produced.

**[Table 7]**

| Component | Mass (%) |
|---|---|
| 1. KSG-42A (Note 1) | 10.0 |
| 2. KSP-101 (Note 2) | 6.0 |
| 3. KF-6104 (Note 3) | 4.0 |
| 4. Disteardimonium Hectorite | 1.5 |
| 5. Silylated Silica | 1.0 |
| 6. TSPL-30-ID (Note 4) | 2.0 |
| 7. Organosiloxane obtained in Production Example 1 (Component (A)) | 5.0 |
| 8. Ethanol | 8.0 |
| 9. Cetiol Ultimate (Note 5) (Component (B)) | 29.0 |
| 10. Isotridecyl Isononanoate | 10.0 |
| 11. KP-578 (Note 6) | 0.5 |
| 12. Titanium Oxide Fine Particles treated by KF-9901 (Note 7) | 8.0 |
| 13. Zinc Oxide Fine Particles treated by KF-9901 (Note 7) | 5.0 |
| 14. Pigment grade Titanium Oxide treated by AES-3083 (Note 8) | appropriate amount |
| 15. Iron Oxide (red) treated by AES-3083 (Note 8) | appropriate amount |
| 16. Iron Oxide (yellow) treated by AES-3083 (Note 8) | appropriate amount |
| 17. Iron Oxide (black) treated by AES-3083 (Note 8) | appropriate amount |
| Total | 100.0 |

| | |
|---|---|
| (Note 1) manufactured by Shin-Etsu Chemical Co., Ltd.: mixture of 75-85% isododecane and 15-25% (vinyl dimethicone/lauryl dimethicone) crosspolymer (Note 2) manufactured by Shin-Etsu Chemical Co., Ltd.: vinyl dimethicone/methicone silsesquioxane crosspolymer (Note 3) manufactured by Shin-Etsu Chemical Co., Ltd.: polyglyceryl-3 polydimethylsiloxyethyl dimethicone (Note 4) manufactured by Shin-Etsu Chemical Co., Ltd.: dissolved product of 30% trimethylsiloxysilylcarbamoyl Pullulan in isododecane (Note 5) manufactured by BASF: mixture of undecane and tridecane (Note 6) manufactured by Shin-Etsu Chemical Co., Ltd.: (acrylates/ethylhexyl acrylate/dimethicone methacrylate) copolymer (Note 7) manufactured by Shin-Etsu Chemical Co., Ltd.: treated by hydrogen dimethicone (Note 8) manufactured by Shin-Etsu Chemical Co., Ltd.: treated by triethoxycaprylylsilane | |

Cetiol Ultimate as the Component 9 was volatile oil, and the kinematic viscosity measured according to the method described above at 25°C was 2.0 mm²/s.

### (Production Method)

A: Components 1 to 9 were uniformly mixed.
B: Components 10 to 17 were uniformly mixed and then subjected to a roll treatment.
C: The product obtained in the above process B were added to the product obtained in the above process A, and the mixture was uniformly mixed.
D: The product obtained by the above process C was defoamed, placed in a container, to obtain oil-based foundation of Example 9.

The inventive oil-based foundation obtained as described above had light spreadability and good fitting to the skin, gave a moist finish, suppressed shine, provided a firm cosmetic film, had good cosmetic persistence, and was extremely excellent.

### (Example 10) Eye Shadow

In accordance with the compounding ratio shown the following Table 8 shown below and production method explained in the below, eye shadow was produced.

**[Table 8]**

| Component | Mass (%) |
|---|---|
| 1. Isododecane (Component (B)) | 25.5 |
| 2. KP-550 (Note 1) | 20 |
| 3. KP-561P (Note 2) | 2 |
| 4. KSP-441 (Note 3) | 6 |
| 5. TMF-1.5 (Note 4) (Component (B)) | 3 |
| 6. Organosiloxane obtained in Production Example 1 (Component (A)) | 5 |
| 7. Vaseline | 5 |
| 8. KSG-320(Note 5) | 5 |
| 9. Amorphous Silicic Acid Anhydride (Note 6) | 1 |
| 10. Barium Sulfate | 5 |
| 11. Organic Pigment | 0.2 |
| 12. KTP-09Y (Note 7) | 1 |
| 13. KTP-09W (Note 7) | 1 |
| 14. Titanated Mica treated by KF-9909 (Note 8) | 20 |
| 15. Tocophenol | 0.2 |
| 16. Fragrance | 0.1 |
| Total | 100 |

| | |
|---|---|
| (Note 1) manufactured by Shin-Etsu Chemical Co., Ltd.: dissolved product of 60% isododecane and 40% acrylates/dimethicone copolymer (Note 2) manufactured by Shin-Etsu Chemical Co., Ltd.: (acrylate/stearyl acrylate/dimethicone methacrylate) copolymer (Note 3) manufactured by Shin-Etsu Chemical Co., Ltd.: polysilicone-22 (Note 4) manufactured by Shin-Etsu Chemical Co., Ltd.: methyl trimethicone (Note 5) manufactured by Shin-Etsu Chemical Co., Ltd.: mixture of 70-80% isododecane and 20-30% (PEG-15/lauryl dimethicone) crosspolymer (Note 6) surface-hydrophobized fumed silica manufactured by Nippon Aerosil Co., Ltd.: AEROSIL R-972 (Note 7) manufactured by Shin-Etsu Chemical Co., Ltd.: KF-9909-treated color inorganic pigment, W: white, Y: yellow (Note 8) manufactured by Shin-Etsu Chemical Co., Ltd.: triethoxysilylethyl polydimethylsiloxyethyl hexyl dimethicone | |

Isododecane as Component 1 was volatile oil, and the kinematic viscosity measured according to the method described above at 25°C was 1.6 mm²/s. Further, TMF-1.5 as Component 5 was volatile oil, and the kinematic viscosity measured according to the method described above at 25°C was 1.5 mm²/s.

### (Production Method)

A: Components 1 to 9 were mixed and uniformly dispersed.
B: Components 10 to 16 were added to the mixture obtained in A and uniformly dispersed to obtain an eye shadow of Example 10.

The eye shadow obtained as described above had good removability, light spreadability, and use feeling with no oily nor powdery touch. It was also verified that the eye shadow had favorable water resistance, water repellency, perspiration resistance, persistence, and difficulty to cause the make-up to smear, and no change due to temperature change and over time, and had excellent stability.

The present description includes the following embodiments.
[1] A cosmetic including:
   (A) organosiloxane represented by the following formula (1), and having a boiling point in a range of 205 to 255°C and a kinematic viscosity at 25°C of less than 5 mm²/s, 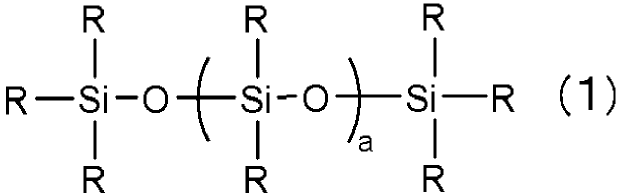 wherein R each independently may be identical to or different from one another and represents a group selected from the group consisting of a hydrogen group, a hydroxy group, and a monovalent hydrocarbon group having 1 to 3 carbon atoms, and "a" is an integer from 1 to 5, provided that at least one of R is a monovalent hydrocarbon group having 2 or 3 carbon atoms; and
   (B) volatile oil having a kinematic viscosity at 25°C of 2 mm²/s or less, excluding the component (A).
[2] The cosmetic according to the above [1], wherein in the general formula (1) of the organosiloxane as the component (A), "a" is 2.
[3] The cosmetic according to the above [1], wherein the organosiloxane as the component (A) is a compound selected from the group consisting of 3,5-diethyl-1,1,1,3,5,7,7,7-octamethyltetrasiloxane, 3,3-diethyl-1,1,1,5,5,7,7,7-octamethyltetrasiloxane, 1,3-diethyl-1,1,3,5,5,7,7,7-octamethyltetrasiloxane, 1,5-diethyl-1,1,3,3,5,7,7,7-octamethyltetrasiloxane, 1,7-diethyl-1,1,3,3,5,5,7,7-octamethyltetrasiloxane, 3,5-dipropyl-1,1,1,3,5,7,7,7-octamethyltetrasiloxane, 3,3-dipropyl-1,1,1,5,5,7,7,7-octamethyltetrasiloxane, 1,3-dipropyl-1,1,3,5,5,7,7,7-octamethyltetrasiloxane, 1,5-dipropyl-1,1,3,3,5,7,7,7-octamethyltetrasiloxane, 1,7-dipropyl-1,1,3,3,5,5,7,7-octamethyltetrasiloxane, and 3-ethyl-5-propyl-1,1,1,3,5,7,7,7-octamethyltetrasiloxane.
[4] The cosmetic according to any one of the above [1] to [3], wherein the volatile oil as the component (B) is at least one compound selected from the group consisting of linear dimethyl silicone oils having a kinematic viscosity at 25°C of less than 2 mm²/s and methyl trimethicone.
[5] The cosmetic according to any one of the above [1] to [4], further including (C) one or more silicone surfactants having a polyether group or a polyglycerin group.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A cosmetic comprising:
(A) organosiloxane represented by the following formula (1), and having a boiling point in a range of 205 to 255°C and a kinematic viscosity at 25°C of less than 5 mm²/s, 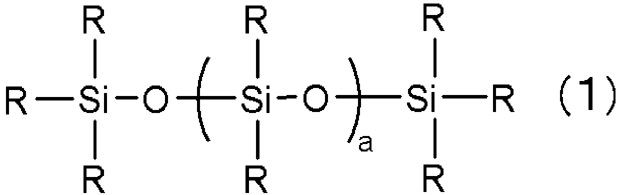 wherein R each independently may be identical to or different from one another and represents a group selected from the group consisting of a hydrogen group, a hydroxy group, and a monovalent hydrocarbon group having 1 to 3 carbon atoms, and "a" is an integer from 1 to 5, provided that at least one of R is a monovalent hydrocarbon group having 2 or 3 carbon atoms; and
(B) volatile oil having a kinematic viscosity at 25°C of 2 mm²/s or less, excluding the component (A).

2. The cosmetic according to claim 1, wherein in the general formula (1) of the organosiloxane as the component (A), "a" is 2.

3. The cosmetic according to claim 1, wherein the organosiloxane as the component (A) is a compound selected from the group consisting of 3,5-diethyl-1,1,1,3,5,7,7,7-octamethyltetrasiloxane, 3,3-diethyl-1,1,1,5,5,7,7,7-octamethyltetrasiloxane, 1,3-diethyl-1,1,3,5,5,7,7,7-octamethyltetrasiloxane, 1,5-diethyl-1,1,3,3,5,7,7,7-octamethyltetrasiloxane, 1,7-diethyl-1,1,3,3,5,5,7,7-octamethyltetrasiloxane, 3,5-dipropyl-1,1,1,3,5,7,7,7-octamethyltetrasiloxane, 3,3-dipropyl-1,1,1,5,5,7,7,7-octamethyltetrasiloxane, 1,3-dipropyl-1,1,3,5,5,7,7,7-octamethyltetrasiloxane, 1,5-dipropyl-1,1,3,3,5,7,7,7-octamethyltetrasiloxane, 1,7-dipropyl-1,1,3,3,5,5,7,7-octamethyltetrasiloxane, and 3-ethyl-5-propyl-1,1,1,3,5,7,7,7-octamethyltetrasiloxane.

4. The cosmetic according to any one of claims 1 to 3, wherein the volatile oil as the component (B) is at least one compound selected from the group consisting of linear dimethyl silicone oils having a kinematic viscosity at 25°C of less than 2 mm²/s and methyl trimethicone.

5. The cosmetic according to any one of claims 1 to 4, further comprising (C) one or more silicone surfactants having a polyether group or a polyglycerin group.
